(19) Europäisches Patentamt
European Patent Office
Office européen des brevets

(11) Veröffentlichungsnummer: 0 293 814
A1

(12) # EUROPÄISCHE PATENTANMELDUNG

(21) Anmeldenummer: 88108657.3

(22) Anmeldetag: 31.05.88

(51) Int. Cl.⁴: C07D 311/92 , C07D 417/12 , A61K 31/35

Patentansprüche für folgende Vertragsstaaten: ES + GR.

(30) Priorität: 03.06.87 DE 3718589

(43) Veröffentlichungstag der Anmeldung: 07.12.88 Patentblatt 88/49

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Lal, Bansi, Dr.
30 Advani Apartments Mulund (West)
Bombay 400 080(IN)
Erfinder: Gangopadhya, Ashok Kumar
B-16, Hoechst Staff Quarters Durga Road
Mulund (West) Bombay 400 082(IN)
Erfinder: Aroskar, Vijay Atmaram
5, Asha Kiran Co op H. Soc. 17-C Linking R. Extn.
Santa Cruz (West) Bombay 400 054(IN)
Erfinder: Dohadwalla, Alihussein Nomanbhai, Dr.
Noman Mansion 139C Cumballa Hill
Bombay 400 036(IN)
Erfinder: Rupp, Richard Helmut, Dr.
Roederweg 16
D-6240 Königstein/Taunus(DE)

(54) **Neue Mono- und Polyhydroxyacyl-Derivate polyoxygenierter Labdane, ein Verfahren zu deren Herstellung und ihre Verwendung als Medikamente.**

(57) Labdanderivate der allgemeinen Formel

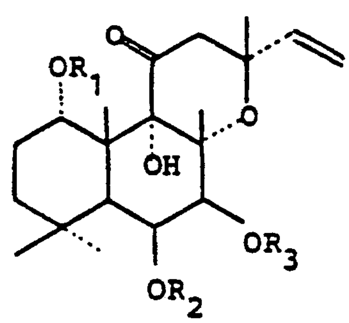

I

den Augeninnendruck senkende pharmazeutische Präparate, die solche Labdanderivate enthalten, Verfahren zur Herstellung der Labdanderivate und die Verwendung der Labdanderivate zur Herstellung der pharmazeutischen Präparate.

EP 0 293 814 A1

# Neue Mono- und Polyhydroxyacyl-Derivate polyoxygenierter Labdane, ein Verfahren zu deren Herstellung und ihre Verwendung als Medikamente

Diese Erfindung betrifft neue Mono- und Polyhydroxyacyl-Derivate polyoxygenierter Labdanderivate und deren Herstellungsverfahren. Die polyoxygenierten Labdanderivate gemäß der Erfindung verfügen über wertvolle pharmakologische Eigenschaften und eignen sich, da sie den Augeninnendruck senken könne, insbesondere zur Behandlung des erhöhten Augeninnendrucks. Die Verbindungen der vorliegenden Erfindung können auch bei Behandlung der dekompensierten Herzinsuffizienz, der Hypertonie und des Bronchialasthmas sowie entzündlicher Erkrankungen eingesetzt werden.

Polyoxygenierte Labdane und deren Derivate sind bereits beschrieben worden in den folgenden Veröffentlichungen: Deutsche Offenlegungsschriften Nr. 25 57 784, 26 40 275, 26 54 796, Europäische veröffentlichte Patentanmeldungen Nr. 0 189 801, 0 217 372, 0 191 166, 0 193 132, Tetrahedron Letters Nr. 19, S. 1669-1672, 1977, J. Chem. Soc., Perkin Trans. 1, 767, 1982.

Gemäß der in den bisherigen Veröffentlichungen genannten pharmakologischen Eigenschaften der polyoxygenierten Labdane und deren Derivate eignen sich diese zur Behandlung von Herz-Kreislauferkrankungen, Hypertonie, Glaukom, Allergie, Bronchokonstriktion und als Immunmodulatoren.

Die Verbindungen gemäß der vorliegenden Erfindung unterscheiden sich von den in den obengenannten Veröffentlichungen beschriebenen dadurch, daß die Mono-oder Polyhydroxyacyloxygruppen nicht nur in 6- und/oder 7-Stellung, sondern auch in 1-Stellung des Labdanmoleküls vorhanden sein können. Es wurde ferner gefunden, daß bestimmte optisch aktive Diastereomere der erfindungsgemäßen Verbindungen eine signifikant stärkere Augeninnendruck-senkende Wirkung aufweisen als die entsprechenden anderen des jeweiligen Diastereomeren-Paares.

Das gilt z. B. auch für die Verbindung 7β-(2,3-Dihydroxypropionyloxy)-8,13-epoxy-1α,6β,9α-trihydroxylabd-14-en-11-on, die in EP-A-O 193 132 als Gemisch der optisch aktiven Diastereomeren beschrieben worden ist.

Die vorliegende Erfindung betrifft polyoxygenierte Labdan-Derivate der Formel I

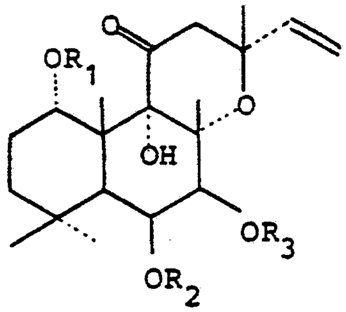

I

worin bedeuten:

$R_1$ Wasserstoff oder einen Rest der Formel

$$-\overset{\displaystyle O}{\overset{\|}{C}}-\underset{\displaystyle R_5}{\underset{|}{(CH)}}{}_n-CH_2-OR_4$$

worin n für 0 oder 1, $R_4$ für Wasserstoff, $C_1$-$C_8$-Alkyl, $C_1$-$C_8$-Alkanoly, Aryl-$C_1$-$C_8$-alkyl oder Aryl und $R_5$ für Wasserstoff; Hydroxy, $C_1$-$C_8$-Alkyl oder Aryl steht, oder $R_4$ und $R_5$ zusammen mit der $-(CH)_n-CH_2-O-$ Gruppe, an die sie gebunden sind, einen Rest der Formel

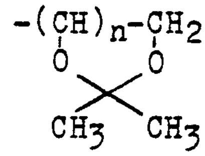

worin n = 1 ist, darstellen,
$R_2$ und $R_3$ gleich oder verschieden sind und Wasserstoff, Acetyl oder einen Rest der Formel

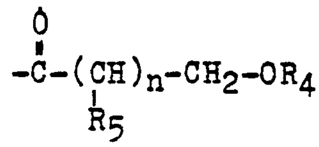

worin $R_4$, $R_5$ und n die oben genannten Bedeutungen haben,
mit der Maßgabe, daß

1. $R_1$, $R_2$ und $R_3$ nicht gleichzeitig Wasserstoff bedeuten,
2. falls $R_1$ und $R_2$ Wasserstoff bedeuten, $R_3$ nicht die Acetylgruppe darstellt, und
3. falls $R_1$ und $R_3$ Wasserstoff bedeuten, $R_2$ nicht die Acetylgruppe darstellt, sowie die optischen und geometrischen Isomere dieser Verbindungen.

Aryl steht für einen Phenylrest, der durch $C_1$-$C_4$-Alkyl, $C_1$-$C_4$-Alkoxy oder Halogen ein- bis dreifach substituiert sein kann. Halogen bedeutet Fluor, Chlor, Brom oder Jod.

Bevorzugte Alkyl- und Alkanoyl-Reste sind solche mit 1 - 6, insbesondere 1 - 4 Kohlenstoffatomen. Ein bevorzugter Aralkylrest ist der Benzylrest, worin das Phenyl wie oben angegeben substituiert sein kann.

Bevorzugt sind Verbindungen der Formel I, worin bedeuten:
$R_1$ Wasserstoff
$R_2$ Wasserstoff oder Acetyl, und
$R_3$ einen Rest der Formel

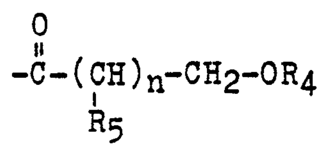

sowie Verbindungen der Formel I, worin bedeuten:
$R_1$ Wasserstoff,
$R_2$ einen Rest der Formel

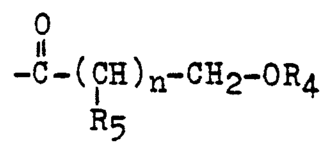

$R_3$ Wasserstoff oder Acetyl,
sowie Verbindungen der Formel I, worin bedeuten:
$R_1$ einen Rest der Formel

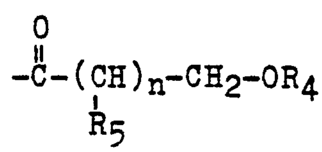

$R_2$ Wasserstoff
$R_3$ Acetyl,

sowie Verbindungen der Formel I, worin bedeuten:
$R_1$ und $R_3$ jeweils einen Rest der Formel

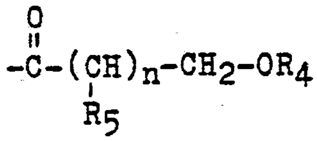

$R_2$ Wasserstoff oder Acetyl, sowie
Verbindungen der Formel I, worin bedeuten:
$R_1$ und $R_2$ einen Rest der Formel

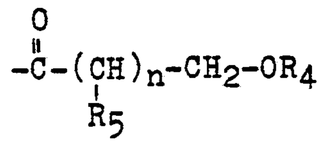

$R_3$ Wasserstoff oder Acetyl,
wobei $R_4$, $R_5$ und n die obengenannten Bedeutungen haben.

Besonders bevorzugt sind die obengenannten Verbindungen der Formel I, worin $R_4$ Wasserstoff, $C_1$-$C_4$-Alkyl, $C_1$-$C_4$-Alkanoyl oder Aryl-$C_1$-$C_4$-alkyl und $R_5$ (falls n = 1) Hydroxy bedeutet.
Ganz besonders bevorzugt sind Verbindungen der Formel I, worin n = 1 ist, $R_4$ Wasserstoff und $R_5$ die Hydroxygruppe bedeutet; und zwar jeweils die R- oder S-Diastereomeren.

In den aufgeführten Formeln ist die Bindung der verschiedenen Substituenten an das Labdangerüst wie folgt gekennzeichnet: eine durchgezogene Linie ( ________ ) markiert einen Substituenten in $\beta$-Stellung (d. h. oberhalb der Ebene des Moleküls) und eine unterbrochene Linie (---) einen Substituenten in $\alpha$-Position (d. h. unterhalb der Ebene des Moleküls). Die Formeln stellen die Substanzen in ihrer absoluten stereochemischen Konfiguration dar. Soweit die Ausgangsmaterialien mit einem Labdangerüst natürlich vorkommen oder von natürlich vorkommenden Materialien abgeleitet sind, besitzen sie ebenso wie die Endprodukte ein Labdangerüst in der einen, hier abgebildeten Konfiguration. Das Verfahren gemäß der vorliegenden Erfindung soll jedoch ebenso für die Synthese von Labdanen der racemischen Serien gelten.

Zusätzlich zu den optischen Zentren des Labdangerüstes können die daran angelagerten Substituenten auch chirale Zentren aufweisen, die zu den optischen Eigenschaften der Substanzen der vorliegenden Erfindung beitragen und Möglichkeiten für deren Auftrennung mittels konventioneller Methoden bieten, beispielsweise durch die Verwendung optisch aktiver Säuren. Eine Wellenlinie (~) als Verbindung einer Gruppe mit einem chiralen Zentrum besagt, daß die Stereochemie des Zentrums unbekannt ist, d. h. die Gruppe kann in jeder der möglichen Stellungen vorliegen. Die vorliegende Erfindung beinhaltet sämtliche optischen Isomere und racemischen Formen der Substanzen der vorliegenden Erfindung, wenn derartige Substanzen zusätzlich zu denen des Labdangerüstes chirale Zentren aufweisen.

Einige der neuen polyoxygenierten Labdanderivate der Erfindung sind in Tabelle 1 aufgeführt.

Tabelle 1

| Verb. Nr. | $R_1$ | $R_2$ | $R_3$ | Fp. |
|---|---|---|---|---|
| 1. | $COCH_2OCH_2C_6H_4$-p-$OCH_3$ | H | $COCH_3$ | |
| 2. | $COCH_2OH$ | H | $COCH_3$ | 196°C |
| 3. | H | H | $CO-CH_2OCH_2CH_3$ | 165-66°C |
| 4. | H | H | $CO-CH_2OC_6H_5$ | 179°C |
| 5. | H | H | $CO_2-CH_2OCH_2C_6H_4$-p-$OCH_3$ | 96°C |
| 6. | H | H | 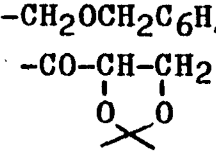 (R) | 173-75°C |
| 7. | | | 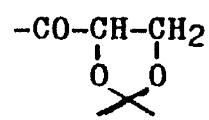 (S) | 191-93°C |
| 8. | | | 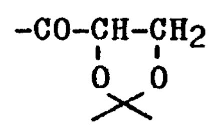 (R,S) | 100°C (Erweichung) |
| 9. | H | H | $COCH_2OH$ | 189°C |
| 10. | H | H | 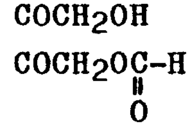 | 199-201°C |
| 11. | H | H | 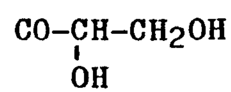 (R) | 166-68°C |

Tabelle 1 (Forts.)

| Verb. Nr. | $R_1$ | $R_2$ | $R_3$ | Fp. |
|---|---|---|---|---|
| 12. | H | H | $CO-\underset{\substack{\vert \\ OH}}{CH}-CH_2OH$ (S) | 198-200°C |
| 13. | H | H | $CO-\underset{\substack{\vert \\ OH}}{CH}-CH_2-OH$ (R,S) | 162-178°C |
| 14. | $-\underset{\substack{\| \\ O}}{C}-CH_2-OCH_2-C_6H_5-p-OCH_3$ | H | $-\underset{\substack{\| \\ O}}{C}-CH_2-O-CH_2-C_6H_4-p-OCH_3$ | Öl |
| 15. | $-\underset{\substack{\| \\ O}}{C}-CH_2-O-\underset{\substack{\| \\ O}}{CH}$ | H | $-\underset{\substack{\| \\ O}}{C}-CH_2-O-\underset{\substack{\| \\ O}}{C}-H$ | 159°C |
| 16. | $-\underset{\substack{\| \\ O}}{C}-CH_2-OC_2H_5$ | H | $-\underset{\substack{\| \\ O}}{C}-CH_2-OC_2H_5$ | 138°C |
| 17. | $-\underset{\substack{\| \\ O}}{C}-CH_2-CH$ | H | $-\underset{\substack{\| \\ O}}{C}-CH_2-OH$ | 102-04°C |
| 18. | $-\underset{\substack{\| \\ O}}{C}-CH_2-OH$ | $-\underset{\substack{\| \\ O}}{C}-CH_2-OH$ | H | 121-23°C |

Tabelle 1 (Forts.)

| Verb. Nr. | $R_1$ | $R_2$ | $R_3$ | Fp. |
|---|---|---|---|---|
| 19. | H | $-\underset{\underset{O}{\Vert}}{C}-CH_2-O-CH_2-C_6H_4-p-OCH_3$ | H | Öl |
| 20. | H | $-\underset{\underset{O}{\Vert}}{C}-CH_2-O-C_6H_5$ | H | 180-182°C |
| 21. | H | $-\underset{\underset{O}{\Vert}}{C}-CH_2-OC_2H_5$ | H | Öl |
| 22. | H | $-\underset{\underset{O}{\Vert}}{C}-CH_2-OH$ | H | 221-23°C |

Die vorliegende Erfindung betrifft auch ein Verfahren zur Herstellung von Verbindungen der Formel I, das dadurch gekennzeichnet ist, daß man eine Verbindung der Formel II

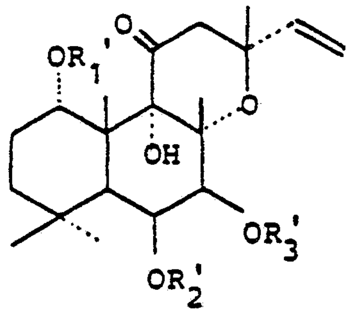

(II)

worin $R_1'$, $R_2'$ und $R_3'$ entweder gleichzeitig Wasserstoff bedeuten oder $R_1'$ und $R_2'$ Wasserstoff bedeuten und $R_3'$ die Acetylgruppe darstellt, umsetzt mit einer Verbindung der Formel III

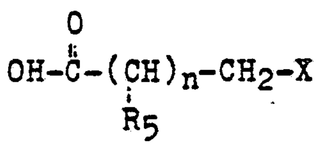

(III)

worin X ein Halogenatom oder den Rest $OR_4$ darstellt, wobei $R_4$, $R_5$ und n die obengenannten Bedeutungen haben, die erhaltenen Verbindungen aus dem Reaktionsgemisch isoliert und reinigt und gegebenenfalls derivatisiert.

Die Acylierung mit einer Verbindung der Formel III wird durchgeführt in organischen Lösungsmitteln wie z. B. Essigsäureethylester in Gegenwart von Carbodiimiden wie z. B. Dicyclohexylcarbodiimid (DCC) und einem Katalysator wie z. B. einem tertiären Amin.

Als tertiäres Amin können beispielsweise 4-Dimethylaminopyridin oder N,N-Dimethylanilin benutzt werden, wobei 4-Dimethylaminopyridin der Vorzug gegeben wird. Die Temperatur, bei der die Acylierungsreaktion erfolgt, ist nicht kritisch, jedoch wird die Reaktion üblicherweise bei einer Temperatur im Bereich von 0 - 50 °C, bevorzugt bei 27 -30 °C ausgeführt. Die Reaktionsdauer beträgt 16 - 24 Stunden, wobei eine Dauer von 16 Stunden am besten geeignet ist. Die Verbindungen der Formel I werden in bekannter Weise aus dem Reaktionsgemisch isoliert. Das Reaktiongsgemisch wird beispielsweise nach dem Filtrieren mit Na-Bikarbonatlösung gewaschen, über wasserfreiem Natriumsulfat getrocknet und dann eingeengt. Der erhaltene Rückstand wird mittels Flash-Chromatographie und anschließend durch Rekristallisation aus organischen Lösungsmitteln wie z. B. einer Petroleumäther:Äthylacetat-Mischung gereinigt.

Die Ausgangsmaterialien 7-Deacetylforskolin und Forskolin sind bekannt (s. Tetrahedron Letters Nr. 19, S. 1669 -1672, 1972, und J. Chem. Soc. Perkin Trans. 1, 767, 1982).

Die Derivatisierung von Verbindungen der Formel I, worin $R_1$ und $R_3$ Wasserstoff und/oder einen Rest der Formel

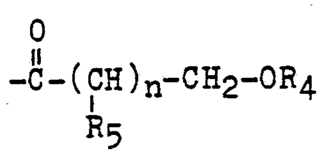

darstellen, worin $R_4$ Aralkyl und n = 0 ist, oder $R_1$ und $R_3$ Wasserstoff und/oder eine Gruppe der Formel

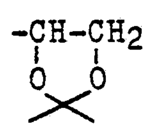

darstellen, erfolgt in üblicher, dem Fachmann bekannter Weise. So wird z. B. eine Verbindung der Formel I, worin $R_1$ den Rest

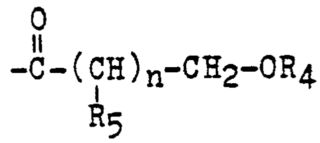

darstellt. $R_4$ für p-Methoxybenzyl steht, n = 0 ist und $R_2$ und $R_3$ Wasserstoff bedeuten, mit dem Reagenz DDQ (2,3-Dichlor-5,6-dicyan-1,4-benzochinon) in einem organischen Lösungsmittel wie Methylenchlorid bei Raumtemperatur zu einer Verbindung der Formel I umgesetzt, worin $R_4$ Wasserstoff bedeutet.

Verbindungen der Formel I, worin $R_3$ eine Gruppe der Formel

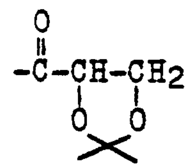

darstellt und $R_1$ und $R_2$ Wasserstoff bedeuten, können mit organischen Säuren wie z. B. p-Toluolsulfonsäure oder Essigsäure in niederen Alkanolen wie Methanol als Lösungsmittel und bei Raumtemperatur umgesetzt werden zu Verbindungen der Formel I, worin $R_3$ den Rest

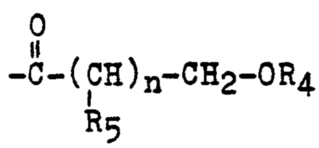

bedeutet, wobei $R_5$ OH und $R_4$ Wasserstoff bedeutet und n = 1 ist.

Die durch Acylierung einer Verbindung der Formel II mit einer Carboxylsäure der Formel

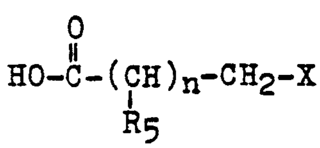

mit X = Halogen, insbesondere Chlor oder Brom, erhaltenen Verbindungen sind bereits bekannt und in EP-A-O 217 372 beschreiben. Durch Derivatisierung dieser Verbindungen gelangt man ebenfalls zu erfindungsgemäßen Verbindungen. So erhält man z. B. durch Behandlung von Verbindungen der Formel I, worin zumindestens einer der Substituenten $R_1$, $R_2$ und $R_3$ den Rest

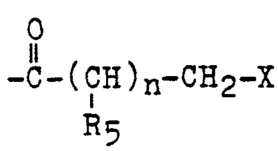

mit X = Halogen darstellt, mit Natriumcarboxylat z. B. Na-formiat in organischen Lösungsmitteln wie Hexamethylphosphorsäureamid bei Raumtemperatur das entsprechende Alkanoyloxyderivat, z. B. das Formyloxyderivat der Formel I.

Verbindungen der Formel I, in denen $R_2$ für Wasserstoff steht, können zur Herstellung der 6-substituierten Verbindungen einer Umlagerungsreaktion unterzogen werden. Die Umlagerung läßt sich ohne Schwierigkeit in wasserlöslichen organischen Lösungsmitteln wie Alkanole oder Acetonitril in Gegenwart von Alkalimethallhydroxid oder Aluminiumoxid vornehmen. An Alkanolen sind Methanol, Äthanol, Isopropanol und t-Butanol zu nennen. Bevorzugt wird allerdings Methanol. Am meisten bevorzugt wird Acetonitril.

Beispiele für Alkalimetallhydroxide sind Natrium-, Kalium- ode Lithiumhydroxid. Bevorzugt wird Natriumhydroxid. Die Temperatur liegt im Bereich von 25 - 30 °C.

Die Labdane der vorliegenden Anmeldung eignen sich zur Behandlung des erhöhten Augeninnendrucks, weil sie, wie mit der Schiotz-Tonometrie nachgewiesen, die Eigenschaft besitzen, den Augeninnendruck herabzusetzen (s. Capriol. J., Sears, M., Lancet, 1, (1983) 958 - 96).

Die Wirksamkeit einiger repräsentativer Verbindungen gemäß der Erfindung ist als prozentuale Senkung des Augeninnendrucks ausgedrückt und in Tab. 2 dargestellt.

Aus den aufgeführten Resultaten ergibt sich zweifelsfrei, daß das optisch aktive Diastereomer (Verbindung Nr. 7 in Tab. 2) den Augeninnendruck wesentlich besser erniedrigt als die anderen Substanzen der Erfindung.

Tabelle 2

| Nr. | Verbindung | Konzentration (%) | Senkung des Augeninnendrucks (%) |
|---|---|---|---|
| 1 | 8,13-Epoxy-7β-(2-äthoxyacetoxy)-1α,6β,9α-trihydroxy-labd-14-en-11-on | 2 | 31 |
| 2 | 8,13-Epoxy-6β-(2-äthoxyacetoxy)-1α,7β,9α-trihydroxy-labd-14-en-11-on | 2 | 20 |
| 3 | 8,13-Epoxy-6β-(2-p-methoxybenzyloxy)-acetoxy-1α,7β,9α-trihydroxy-labd-14-en-11-on | 2 | 31 |
| 4 | 8,13-Epoxy-7β-(2-formyloxyacetoxy)-1α,6β,9α-trihydroxy-labd-14-en-11-on | 2 | 31 |
| 5 | 8,13-Epoxy-1α,7β-bis(2-formyloxyacetoxy)-6β,9α-dihydroxy-labd-14-en-11-on | 2 | 26 |
| 6 | 8,13-Epoxy-1α,7β-bis(2-hydroxyacetoxy)-6β,9α-dihydroxy-labd-14-en-11-on | 2 | 24 |
| 7 | 7β-(2R,3-Dihydroxy-propionyloxy)-8,13-epoxy-1α,6β,9α-trihydroxy-labd-14-en-11-on | 2<br>0,5<br>0,25<br>0,1 | 27<br>25<br>24<br>17 |
| 8 | 7β-(2S,3-Dihydroxypropionyloxy)-8,13-epoxy-1α,6β,9α-trihydroxy-labd-14-en-11-on | 2<br>0,5<br>0,25 | |
| 9 | 7β-(2R,S,3-Dihydroxypropionyloxy)-8,13-epoxy-1α,6β,9α-trihydroxy-labd-14-en 11-on | 2<br>0,5 | 26,7<br>19 |

Für die Behandlung des erhöhten Augeninnendrucks, z. B. beim Glaukom, werden die erfindungsgemäßen Verbindungen topisch angewendet, und zwar üblicherweise zusammen mit einem physiologisch verträglichen Trägerstoff, z. B. wäßriger Methylcellulose. Arnzeimittel mit entsprechender Wirkung, welche die erfindungsgemäßen Verbindungen enthalten, können auch in Form von Suspensionen, Lösungen, Emulsionen oder Salben verwendet werden. Bevorzugt sind Zubereitungen, welche eine längerdauernden Kontakt mit dem Auge und/oder ein schnelleres Eindringen des Wirkstoffs in das Auge bewirken, z. B. durch Kombination mit Benzalkoniumchlorid.

Für die topische Anwendung am Auge haben sich Konzentrationen von 0,1 bis 4, bevorzugt 1 bis 4 %, einer der erfindungsgemäßen Verbindungen in der Zubereitung als wirksam erwiesen. Die Häufigkeit der Anwendung richtet sich nach der Konzentration des Wirkstoffs; im allgemeinen werden bei Zubereitungen mit der genannten Konzentration 3 - 4 Verabreichungen pro Tag genügen.

**Beispiel 1**

8,13-Epoxy-7β,2-äthoxyacetoxy-1α,6β,9α-trihydroxy-labd-14-en-11-on

8,13-Epoxy-1α,6β,7β,9α-tetrahydroxy-labd-14-en-11-on (1 mol) wurde zu einer gut umgerührten Mischung aus 2-Äthoxyessigsäure (1,2 mol), Dicyclohexylcarbodiimid (1,32 mol) und 4-Dimethylaminopyridin (1,2 mol) in Äthylacetat bei Zimmertemperatur zugesetzt. Das Rühren wurde bei Zimmertemperatur 16 Stunden fortgesetzt. Dann wurde das Reaktionsgemisch filtriert und das Filtrat mit verdünnter Natriumbikarbonatlösung und Kochsalzlösung gewaschen. Die organische Schicht wurde über wasserfreiem Natriumsulfat getrocknet und dann unter reduziertem Druck konzentriert.

Der gewonnene Rückstand wurde mittels flash-Säulenchromatographie über Keiselgel gereinigt. Man erhält das reine Produkt mit einem Schmelzpunkt von 165-66° C und einer Ausbeute von 76 %.

Analyse:

Berechnet für $C_{24}H_{38}O_8$:
C% 63,41; H% 8,43
gefunden:
C% 63,43; H% 8,33

Gemäß dem oben beschriebenen Verfahren und unter Verwendung der entsprechenden Säure anstelle von 2-Äthoxyessigsäure wurden die folgenden Substanzen hergestellt:

7β-Acetoxy-8,13-epoxy-1α-(2-p-methoxybenzyloxy)-acetoxy-6β,9α-dihydroxy-labd-14-en-11-on; Öl, Ausbeute 41 %

8,3-Epoxy-7β-2-phenoxyacetoxy-1α,6β,9α-trihydroxy-labd-14-en-11-on; Schmelzpunkt 179° C, Ausbeute 82%

8,13-Epoxy-7β-(2-p-methoxybenzyloxy)-acetoxy-1α,6β,9α-trihydroxy-labd-14-en-11-on; Schmelzpunkt 96° C, Ausb. 96 %

7β-(2R,3-O-Isopropylidinopropionyloxy)-8,13-epoxy-1α,6β,9α-trihydroxy-labd-14-en-11-on; Schmelzpunkt 173 - 75° C, Ausbeute 51 %

8β-(2S,3-O-Isopropylidinopropionyloxy)-8,13-epoxy-1α,6β,9α-trihydroxy-labd-14-en-11-on; Schmelzpunkt 191 - 93° C

7β-(2,3-O-Isopropylidinopropionyloxy)-8,13-epoxy-1α,6β,9α-trihydroxy-labd-14-en-11-on; Schmelzpunkt 100° C (Erweichung), Ausbeute 50 %

1α,7β-Bis-(2-p-methoxybenzyloxy)-acetoxy-6β,9α-dihydroxy-labd-14-en-11-on (Öl), Ausbeute 8 %

1α,7β-Bis-(2-Äthoxyacetoxy-6β,9α-dihydroxy-labd-14-en-11-on; Schmelzpunkt 138° C, Ausbeute 11 %

**Beispiel 2**

7β-Acetoxy-6β,9α-dihydroxy-8,13-epoxy-1α-(2-hydroxyacetoxy)-labd-14-en-11-on

7β-Acetoxy-6β,9α-dihydroxy-8,13-epoxy-1α-(2-p-methoxybenzyloxy)-acetoxy-labd-14-en-11-on (5 mmol) wurde in Dichlormethan (140 ml) gelöst, dann wurde Wasser (16 ml) und 2,3-Dichlor-5,6-dicyano-1,4-benzochinon (7,5 mmol) unter starkem Rühren zugefügt. Das Reaktionsgemisch wurde bei Zimmertemperatur 16 Stunden lang gerührt und dann fitriert. Das Filtrat wurde mit wäßriger Natriumdithionat-Lösung und anschließend mit Kochsalzlösung gewaschen. Die organische Schicht wurde über wasserfreiem Natriumsulfat getrocknet, das Lösungsmittel unter Vakuum entfernt. Der gewonnene Rückstand wurde mittels flash-Chromatographie über Kieselgel mit 30 % Acetonitril in Chloroform als Eluent gereinigt. Ausbeute 82,8 %, Schmelzpunkt 196 °C (aus Äthylacetat/Petroleumäther-Mischung kristallisiert).

Analyse:

Berechnet für $C_{22}H_{34}O_8$:
C% 61,95, H% 8,03
Gefunden:
C% 61,76, H% 7,69

Nach dem oben beschriebenen Verfahren und unter Verwendung des geeigneten Labdanderivates wurden die folgenden Substanzen hergestellt:

8,13-Epoxy-7β-(2′-Hydroxyacetoxy)-1α,6β,9α-trihydroxy-labd-14-en-11-on; Schmelzpunkt 189 °C, Ausbeute 63 %

1α,7β-bis-(2-Hydroxyacetoxy)-6β,9α-dihydroxy-8,13-epoxy-labd-14-en-11-on; Schmelzpunkt 102 - 104 °C, Ausbeute 30 %

**Beispiel 3**

7β-(2R,3-Dihydroxypropionyloxy)-8,13-epoxy-1α,6β,9α-trihydroxy-labd-14-en-11-on, Hemihydrat

7β-(2R-3-O-Isopropylidinopropionyloxy)-8,13-epoxy-1α,6β,9α-trihydroxy-labd-14-en-11-on(0,4 mol) wurde in Methanol (6 ml) gelöst. Zu dieser Lösung wurde unter kräftigem Rühren p-Toluolsulfonsäure (0,8 mol) zugesetzt und das Rühren bei Zimmertemperatur 2 Stunden fortgesetzt. Das Methanol wurde dann aus dem Reaktionsgemisch destilliert und der Rückstand in Äthylacetat gelöst. Die Äthylacetatlösung wurde mit verdünnter wäßriger Natriumbikarbonatlösung und anschließend mit Kochsalzlösung gewaschen. Die organische Schicht wurde abgetrennt, über wasserfreienm Natriumsulfat getrocknet und konzentriert. Der gewonnene Rückstand wurde mittels flash-Chromatographie über Kieselgel unter Verwendung von 20 % Acetonitril in Chloroform als Eluent gereinigt. Ausbeute 74,67 %, Schmelzpunkt 166 - 68 °C (aus Äthylacetat/Petroleumäther-Mischung kristallisiert).

Analyse

Berechnet für $C_{23}H_{36}O_9 \bullet H_2O$:
C% 59,35, H% 7,95
Gefunden:
C% 59,06, H% 7,64

Nach dem oben beschriebenen Verfahren und mit geeignetem Labdanderivat als Ausgangsmaterial wurden folgende Substanzen hergestellt:

7β-(2S,3-Dihydroxypropionyloxy)-8,13-epoxy-1α,6β,9α-trihydroxy-labd-14-en-11-on, Schmelzpunkt 198 - 200 °C

7β-(2,3-Dihydroxypropionyloxy)-8,13-epoxy-1α,6β,9α-trihydroxy-labd-14-en-11-on, Schmelzpunkt 162 - 178 °C, Ausbeute 45 %

**Beispiel 4**

8,13-Epoxy-7β-(2-formyloxyacetoxy)-1α,6β,9α-trihydroxy-labd-14-en-11-on

7β-(2-Bromacetoxy)-1α,6β,9α-trihydroxy-8,13-epoxy-labd-14-en-11-on (1 mmol) wurden in Hexamethylphosphoramid (6 ml) gelöst. Dieser Lösung wurde wasserfreies Natriumformiat (2 mmol) unter Rühren bei Zimmertemperatur zugesetzt und weitere 16 Stunden lang gerührt. Das Hexamethylphosphoramid wurde dann unter vermindertem Druck herausdestilliert, der Rückstand mit Diäthyläther extrahiert. Der ätherische Extrakt wurde mit Kochsalzlösung gewachen, über wasserfreiem Natriumsulfat getrocknet und im Vakuum konzentriert. Der gewonnene Rückstand wurde mittels flash-Chromatographie über Kieselgel untr Verwendung von 5 % Acetonitril in Chloroform als Eluent gereinigt.
Ausbeute: 94,5 %, Schmelzpunkt 199 - 201 °C (aus Äthylacetat/Petroleumäther-Mischung kristallisiert).

Analyse:

Berechnet für $C_{23}H_{34}O_9$:
C% 60,78, H% 7,54
Gefunden:
C% 60,38, H% 7,36

Nach dem oben beschriebenen Verfahren wurde 1α,7β-Bis-(2-Bromacetoxy)-6β,9α-dihydroxy-8,13-epoxy-labd-14-en-11-on zu 1α, 7β-Bis-(2-formyloxyacetoxy)-6β,9α-dihydroxy-8,13-epoxy-labd-14-en-11-on umgewandelt. Ausbeute 94 %, Schmelzpunkt 159 °C (aus Äthylacetat/Petroleumäther kristallisiert).

**Beispiel 5**

8,13-Epoxy-6β-(2-äthoxyacetoxy)-1α,7β,9α-trihydroxy-labd-14-en-11-on

8,13-Epoxy-7β-(2-äthoxyacetoxy)-1α,6β,9α-trihydroxy-labd-14-en-11-on (1 mol) wurde in Acetonitril (17 ml) und Wasser (21 ml) gelöst. Dieser Mischung wurde unter kräftigem Rühren bei Zimmertemperatur Natriumhydroxid-Lösung (1N, 1 ml) zugesetzt. Nach halbstündigem Rühren wurde das Reaktionsgemisch mit verdünnter Salzsäure auf pH 7 neutralisiert und anschließend bei vermindertem Druck konzentriert. Der gewonnene Rückstand wurde mit Äther extrahiert, der ätherische Extrakt mit Kochsalzlösung gewaschen undüber wasserfreiem Natriumsulfat getrocknet. Nach Konzentrieren des Extraktes und Reinigung des Rückstandes mittels flash-Chromatographie auf der Kieselgelsäule unter Verwendung von 10 % Acetonitril in Chloroform als Eluent betrug die Ausbeute des Produktes (als Schaum) 29 %.

Analyse:

Berechnet für $C_{24}H_{38}O_8$:
C% 63,41, H% 8,42
Gefunden:
C% 63,64, H% 8,55

Auf die gleiche Weise wurden aus den entrsprechenden 7-substituierten polyoxygenierten Derivaten die folgenden Substanzen hergestell.
8,13-Epoxy-6β-phenyloxyacetoxy-1α,7β,9α-trihydroxy-labd-14-en-11-on; Schmelzpunkt 180 - 82 °C, Ausbeute 31 %
8,13-Epoxy-6β-2-(p-methoxybenzyloxy)-acetoxy-1α,7β,9α-trihydroxy-labd-14-en-11-on (Öl), Ausbeute 33 %

**Beispiel 6**

8,13-Epoxy-6β-(2-hydroxyacetoxy)-1α,7β,9α-trihydroxy-labd-14-en-11-on

8,13-Epoxy-7β-(2-formyloxyacetoxy)-1α,6β,9α-trihydroxy-labd-14-en-11-on (0,44 mol) wurde in Methylenchlorid mit 1 % Methanol (20 ml) gelöst und durch eine Aluminiumoxidsäule (29 g) passiert, die mit Methylenchlorid und 1 % Methanol beschickt war. Durch Elution mit Methylenchlorid und 5 % Methanol (500 ml) und Einengen des Eluenten unter vermindertem Druck wurde der Rückstand gewonnen. Die Reinigung des Rückstandes erfolgt mittels flash-Chromatographie unter Verwendung von 20 % Acetonitril in Chloroform, die Produktausbeute betrug 80 %, der Schmelzpunkt 221 - 23 °C.

Analyse:

Berechnet für $C_{22}H_{34}N_8$:
C% 61,95, H% 8,03
Gefunden:
C% 62,27, H% 7,92

Nach dem gleichen Verfahren wurde mit 1α,7β-Bis-(2-formyloxyacetoxy)-6β,9α-dihydroxy-8, 13-epoxy-labd-14-en-11-on das Produkt 1α,6β-Bis-(2-Hydroxyacetoxy)-7β,9α-dihydroxy-8,13-epoxy-labd-14-en-11-on mit einer Ausbeute von 67 % und einem Schmelzpunkt von 121 - 123 °C hergestellt.

**Ansprüche**

1. Verbindungen der Formel I

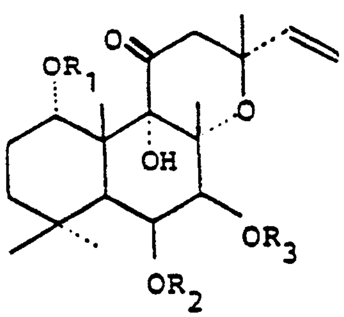

(I)

worin bedeuten:
$R_1$ Wasserstoff oder einen Rest der Formel

$$-\overset{O}{\overset{\|}{C}}-(\underset{R_5}{\underset{|}{CH}})_n-CH_2-OR_4$$

worin n für 0 oder 1, $R_4$ für Wasserstoff, $C_1$-$C_8$-Alkyl, $C_1$-$C_8$-Alkanoyl, Aryl-$C_1$-$C_8$-alkyl oder Aryl und $R_5$ für Wasserstoff, Hydroxy, $C_1$-$C_8$-Alkyl oder Aryl steht, oder $R_4$ und $R_5$ zusammen mit der $-(CH)_n-CH_2-O-$ Gruppe, an die sie gebunden sind, einen Rest der Formel

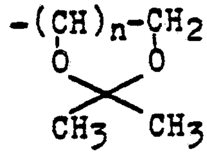

worin n = 1 ist, darstellen,
$R_2$ und $R_3$ gleich oder verschieden sind und Wasserstoff, Acetyl oder einen Rest der Formel

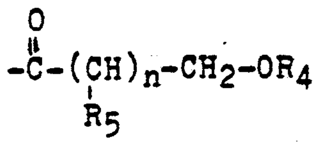

worin $R_4$, $R_5$ und n die oben genannten Bedeutungen haben, bedeuten,
mit der Maßgabe, daß
1. $R_1$, $R_2$ und $R_3$ nicht gleichzeitig Wasserstoff bedeuten,
2. falls $R_1$ und $R_2$ Wasserstoff bedeuten, $R_3$ nicht die Acetylgruppe darstellt, und
3. falls $R_1$ und $R_3$ Wasserstoff bedeuten, $R_2$ nicht die Acetylgruppe darstellt,
sowie die optischen und geometrischen Isomere dieser Verbindungen.

2. Verbindungen der Formel I gemäß Anspruch 1, worin bedeuten:
$R_1$ Wasserstoff,
$R_2$ und $R_3$ verschieden sind und Wasserstoff oder einen Rest der Formel

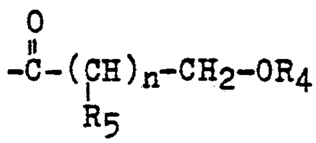

bedeuten, worin $R_4$ und $R_5$ und n die genannten Bedeutungen haben.

3. Verbindungen der Formel I gemäß Anspruch 2, worin $R_4$ Wasserstoff, $C_1$-$C_4$-Alkyl, $C_1$-$C_4$-Alkanoyl, Aryl oder Aryl-$C_1$-$C_4$-alkyl und $R_5$, falls n = 1 ist, Hydroxy bedeutet.

4. Verbindungen der Formel I gemäß Anspruch 3, worin $R_4$ Methyl, Äthyl, Formyl oder Acetyl, Phenyl, Benzyl, p-Methoxybenzyl oder, falls n = 1 ist, Wasserstoff bedeutet.

5. Verfahren zur Herstellung von Verbindungen gemäß Anspruch 1, dadurch gekennzeichnet, daß man eine Verbindung der Formel II

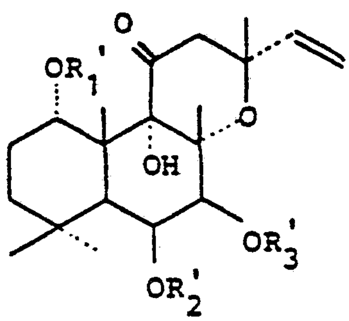

(II)

worin $R'_1$, $R'_2$ und $R'_3$ entweder gleichzeitig Wasserstoff bedeuten, oder $R'_1$ und $R'_2$ Wasserstoff und $R'_3$ die Acetylgruppe darstellen, umsetzt mit einer Verbindung der Formel III

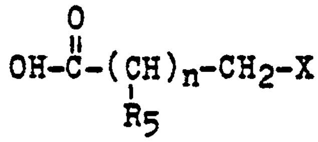

(III)

worin X Halogen oder den Rest $-OR_4$ bedeutet, wobei $R_4$, $R_5$ und n die obengenannten Bedeutungen haben, die gebildeten Verbindungen aus dem Reaktionsgemisch isoliert und sie gegebenenfalls nach üblichen Methoden derivatisiert.

6. Arzneimittel, gekennzeichnet durch einen Gehalt an einer Verbindung gemäß Anspruch 1 und üblichen Hilfs- und Trägerstoffen.

7. Verwendung einer Verbindung gemäß Anspruch 1 zur Herstellung von Arzneimitteln mit Augeninnendrucksenkender Wirkung.

Patentansprüche für die folgenden Vertragsstaaten: GR, ES

1. Verfahren zur Herstellung von Verbindungen der Formel I

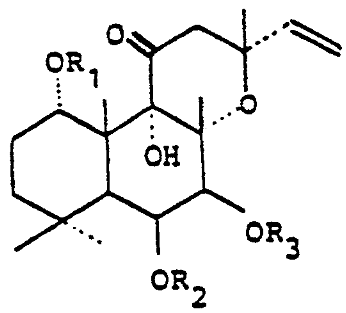

(I)

worin bedeuten:
$R_1$ Wasserstoff oder einen Rest der Formel

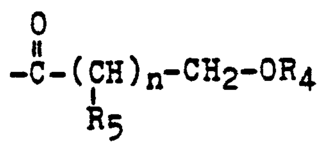

worin n für 0 oder 1, $R_4$ für Wasserstoff, $C_1$-$C_8$-Alkyl, $C_1$-$C_8$-Alkanoyl, Aryl-$C_1$-$C_8$-alkyl oder Aryl und $R_5$ für Wasserstoff, Hydroxy, $C_1$-$C_8$-Alkyl oder Aryl steht, oder $R_4$ und $R_5$ zusammen mit der $-(CH)_n-CH_2-O-$ Gruppe, an die sie gebunden sind, einen Rest der Formel

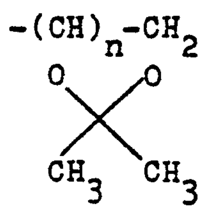

darstellen,
$R_2$ und $R_3$ gleich oder verschieden sind und Wasserstoff, Acetyl oder einen Rest der Formel

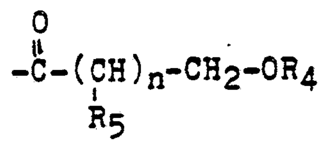

worin $R_4$, $R_5$ und n die oben genannten Bedeutungen haben, bedeuten,
mit der Maßgabe, daß
1. $R_1$, $R_2$ und $R_3$ nicht gleichzeitig Wasserstoff bedeuten,
2. falls $R_1$ und $R_2$ Wasserstoff bedeuten, $R_3$ nicht die Acetylgruppe darstellt, und
3. falls $R_1$ und $R_3$ Wasserstoff bedeuten, $R_2$ nicht die Acetylgruppe darstellt,
sowie die optischen und geometrischen Isomeren dieser Verbindungen,
dadurch gekennzeichnet, daß man eine Verbindung der Formel II

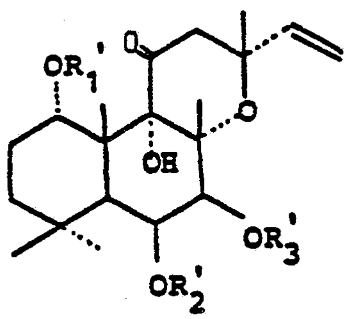

(II)

worin $R'_1$, $R'_2$ und $R'_3$ entweder gleichzeitig Wasserstoff bedeuten, oder $R'_1$ und $R'_2$ Wasserstoff und $R'_3$ die Acetylgruppe darstellen, umsetzt mit einer Verbindung der Formel III

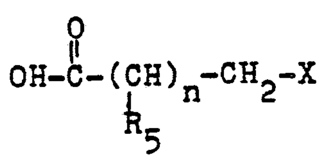

(III)

worin X Halogen oder den Rest $-OR_4$ bedeutet, wobei $R_4$, $R_5$ und n die obengenannten Bedeutungen haben, die gebildeten Verbindungen aus dem Reaktionsgemisch isoliert und sie gegebenenfalls nach üblichen Methoden derivatisiert.

Europäisches Patentamt

# EUROPÄISCHER RECHERCHENBERICHT

Nummer der Anmeldung

EP 88108657.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 252 482 (HOECHST AKTIENGESELLSCHAFT)<br>* Ansprüche 1,6,8-9 *<br>-- | 1,5,6,7 | C 07 D 311/92<br>C 07 D 417/12<br>A 61 K 31/35 |
| A | WO - A1 - 85/02 616 (BROWN UNIVERSITY RESEARCH FOUNDATION)<br>* Ansprüche 1,6 *<br>-- | 1,5,6,7 | |
| A | EP - A2 - 0 222 413 (NIPPON KAYAKU KABUSHIKI KAISHA)<br>* Zusammenfassung *<br>-- | 1,5-7 | |
| D,A | EP - A2 - 0 217 372 (HOECHST AKTIENGESELLSCHAFT)<br>* Ansprüche 1,6,7 *<br>-- | 1,5-7 | RECHERCHIERTE SACHGEBIETE (Int. Cl.4) |
| D,A | EP - A1 - 0 189 801 (HOECHST AKTIENGESELLSCHAFT)<br>* Anspruch 1 *<br>---- | 1,5-7 | C 07 D 311/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 13-09-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82